# EUROPÄISCHE PATENTSCHRIFT

(11) **EP 0 963 977 B1**
(45) Veröffentlichungstag und Bekanntmachung des Hinweises auf die Patenterteilung: **07.05.2003**
(21) Anmeldenummer: 99110790.5
(22) Anmeldetag: 04.06.1999
(51) Int. Cl.: C07D 215/56, C07D 471/04

(54) **Verfahren zur Herstellung von Chinolon-und Naphthyridoncarbonsäuren und deren Ester**
Process for the preparation of quinolone- and naphthyridonecarboxylic acids and their esters
Procédé pour la préparation des acides quinolone- et naphtyridonecarboxyliques et de leurs esters

(30) Priorität: 12.06.1998 DE 19826050
(43) Veröffentlichungstag der Anmeldung: 15.12.1999
(73) Patentinhaber: Bayer Aktiengesellschaft, 51368 Leverkusen (DE)
(72) Erfinder: Lui, Norbert Dr., 51061 Köln (DE); Panskus, Hans, 51373 Leverkusen (DE); Müller, Herbert Dr., 52372 Kreuzau-Bilstein (DE)

(56) Entgegenhaltungen:
- EP-A- 0 176 846
- EP-A- 0 300 311
- WO-A-97/31001
- PATENT ABSTRACTS OF JAPAN vol. 17, no. 361 (C-1080), 8. Juli 1993 (1993-07-08) & JP 05 051365 A (UBE IND LTD), 2. März 1993 (1993-03-02)

## Beschreibung

Die vorliegende Erfindung betrifft ein verbessertes Verfahren zur Herstellung von Chinolon- und Naphthyridoncarbonsäuren und deren Ester, ausgehend von Benzoesäurechloriden bzw. Nicotinsäurechloriden.

Chinolon- und Naphthyridoncarbonsäuren und deren Ester sind Zwischenprodukte für die Herstellung bekannter, pharmazeutisch wirksamer Chinoloncarbonsäuren bzw. Naphthyridoncarbonsäuren.

Aus der EP-A-300 311 ist eine Herstellung von Chinoloncarbonsäuren bekannt, bei der man ein Benzoesäurechlorid mit einem Aminoacrylsäureester acyliert, beim erhaltenen Aroylacrylester einen Aminaustausch durchführt, das erhaltene Aminoacrylat cyclisiert, den erhaltenen Ester verseift und die erhaltene Chinoloncarbonsäure durch Zugabe einer Säure ausfällt. Die Ausbeuten liegen dabei zwischen 71 und 79 %. Als Lösungsmittel fiir die einzelnen Reaktionsstufen werden angegeben: Für die Acylierung Toluol, Xylol, Cyclohexan, offenkettige Kohlenwasserstoffe, DMF und DMSO, für den Aminaustausch zusätzlich Alkohole und Butylglykol und für die Cyclisierung nur höhere Alkohole, Aminoalkohole, DMF, DMSO, Dioxan und N-Methylpyrrolidon.

Wenn man für die Acylierung und den Aminaustausch wenig polare bis unpolare Lösungsmittel, z.B. Kohlenwasserstoffe einsetzen möchte, dann muß man für die Cyclisierung ein anderes, polares, gegebenenfalls auch protisches Lösungsmittel einsetzen, z.B. Butylalkohol. Die Durchführung der gesamten Reaktion in nur einem Lösungsmittel erscheint allenfalls in einem stark polaren Lösungsmittel, z.B. DMF oder DMSO möglich. Bei allen Beispielen der EP-A 300 311 ist ein Lösungsmittelwechsel vorgenommen worden, nämlich vom unpolaren, aprotischen Toluol oder Cyclohexan für die erste und gegebenenfalls zweite Stufe zum polaren, protischen Butylglykol für die dritte und gegebenenfalls zweite Stufe.

Der Wechsel des Lösungsmittels führt zu erheblichen Aufwand für die separate Abtrennung von zwei verschiedenen Lösungsmitteln, die Trocknung des Zwischenprodukts, auf dessen Stufe der Lösungsmittelwechsel erfolgt und die Entsorgung bzw. Aufbereitung von zwei verschiedenen Lösungsmitteln. Außerdem sind die erzielbaren Ausbeuten noch nicht voll zufriedenstellend.

Gemäß der EP-A 176 846 wird fiir die Umsetzung eines Benzoylhalogenids mit einem Acrylsäurederivat (= Acylierung) Methylenchlorid, Chloroform, Toluol, Tetrahydrofuran oder Dioxan verwendet.

In Liebigs Ann. Chem. 1987, 29-37 wird für die Cyclokondensation von 3-Amino-2-benzoylacrylsäureestern zu 4-Chinolon-3-carbonsäureestern (= Cyclisierung) ein dipolares, aprotisches Lösungsmittel, z.B. DMF, DMSO oder N-Methylpyrrolidon vorgeschrieben.

Es besteht also ein allgemeines technisches Vorurteil dagegen, für die gesamte Reaktionssequenz ein wenig polares bis unpolares Lösungsmittel zu verwenden.

Es wurde nun ein Verfahren zur Herstellung von Chinolon- und Naphthyridoncarbonsäuren und deren Ester der Formel (I) gefunden 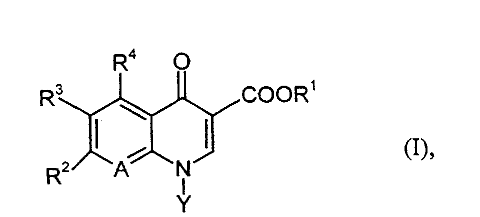 in der
- R¹: für Wasserstoff oder C₁-C₄-Alkyl,
- R²: für Halogen,
- R³: für Halogen,
- R⁴: für Wasserstoff, Halogen oder Nitro,
- Y: für C₁-C₆-Alkyl, 2-Fluorethyl, Cyclopropyl, Fluorcyclopropyl, Isopropyl, 4-Fluorphenyl oder 2,4-Difluorphenyl und
- A: für Stickstoff oder C-R⁵ mit R⁵ = Wasserstoff, Methyl, Methoxy, Halogen, Nitro oder Cyano stehen,
wobei Y und R⁵ auch gemeinsam -CH₂-CH₂-O- oder -CH(CH₃)-CH₂-O- bedeuten können, wobei die endständige CH₂- bzw. die CH(CH₃)-Gruppe an das Stickstoffatom gebunden ist,
das dadurch gekennzeichnet ist, daß man
a) ein Benzoyl- oder Nicotinoylchlorid der Formel (II) 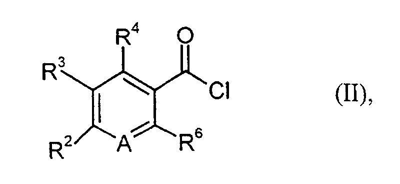 in der
   - R², R³, R⁴ und A: die bei Formel (I) angegebene Bedeutung haben und
   - R⁶: für Halogen steht,
   in Gegenwart einer Base mit einem Aminoacrylsäureester der Formel (III) 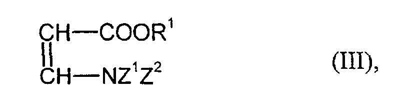 in der
   - R¹: für C₁-C₄-Alkyl und
   - Z¹ und Z²: unabhängig voneinander für C₁-C₄-Alkyl stehen oder zusammen mit dem Stickstoffatom, an das sie gebunden sind, einen 5- bis 6-gliedrigen gesättigten oder ungesättigten Ring bilden, der gegebenenfalls bis zu zwei weitere Heterogruppen aus der Gruppe O-Atome, S-Atome und SO₂-Gruppen enthalten kann,
   umsetzt und so einen (Het)-Aroylacrylester der Formel (IV) erhält 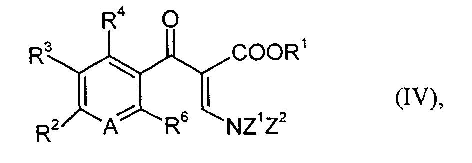 in der
   - R¹: für C₁-C₄-Alkyl steht und
   - R², R³, R⁴ und A: die bei Formel (I) angegebene,
   - R⁶: die bei Formel (II) angegebene und
   - Z¹ und Z²: die bei Formel (III) angegebene Bedeutung haben,
b) den (Het-)Aroylacrylester der Formel (IV) mit einem Amin der Formel (V)

   H₂N-Y (V),

   in der
   - Y: die bei Formel (I) angegebene Bedeutung hat,
   einem Aminaustausch unterwirft und so einen (Het-)Aroylacrylester der Formel (VI) erhält 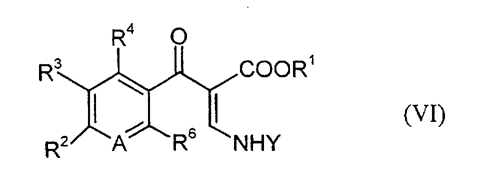 in der
   - R¹: für C₁-C₄-Alkyl steht und
   - R², R³, R⁴, Y und A: die bei Formel (I) angegebene und
   - R⁶: die bei Formel (II) angegebene Bedeutung haben,
c) den (Het-)Aroylacrylester der Formel (VI) in Gegenwart einer Base cyclisiert und so einen Chinolon- bzw. Naphthyridonester der Formel (I) erhält, bei dem R¹ für C₁-C₄-Alkyl steht und
d) falls man eine Chinolon- oder Naphthyridoncarbonsäure der Formel (I), in der R¹ für Wasserstoff steht, herstellen möchte, den nach der Stufe c) vorliegenden Ester verseift und die Säure der Formel (I), in der R¹ für Wasserstoff steht, nach Zugabe einer Säure isoliert,
wobei man die Zwischenprodukte der Formeln (IV) und (VI) nicht isoliert und die Stufen a) bis c) in Gegenwart des gleichen, wenig polaren bis unpolaren Lösungsmittels durchführt.

In den Formeln (I) bis (VI) verwendeten Symbole haben vorzugsweise folgende Bedeutungen:

Soweit R¹ für C₁-C₄-Alkyl steht: Methyl oder Ethyl.
- R²:: Chlor oder Fluor.
- R³:: Fluor.
- R⁴:: Wasserstoff, Chlor, Fluor oder Nitro.
- R⁶:: Fluor oder Chlor.
- A:: C-R⁵ mit R⁵ = Wasserstoff, Methyl, Methoxy, Halogen oder Cyano oder N.
- Y:: Ethyl, Cyclopropyl, Fluorcyclopropyl, 2,4-Difluorphenyl oder zusammen mit R⁵ -CH(CH₃)-CH₂-O-.
- Z¹ und Z²:: Methyl oder Ethyl.

Für die Stufe a) kommen beispielsweise Reaktionstemperaturen im Bereich 25 bis 120°C in Frage. Bevorzugt arbeitet man bei 30 bis 80°C. Als Basen für die Stufe a) kommen beispielsweise tertiäre Amine in Frage, wie solche der Formeln 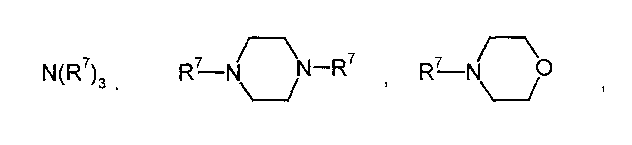 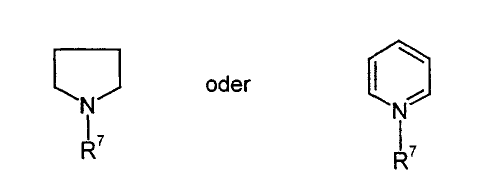 bei denen R⁷ für C₁-C₁₄-Alkyl oder Benzyl steht.

Falls mehrere R⁷-Gruppen in einem Molekül vorhanden sind, können diese gleich oder verschieden sein. Bevorzugt steht R⁷ für C₁-C₄-Alkyl. Besonders bevorzugt als tertiäres Amin ist Triethylamin.

In die Stufe a) wird im allgemeinen mindestens ein Äquivalent Base bezogen auf 1 Mol des Säurechlorids der Formel (II) eingesetzt. Bevorzugt beträgt diese Menge 1 bis 2 Äquivalente. Größere Mengen sind nicht kritisch, aber unwirtschaftlich.

Während der Reaktion ausfallendes Hydrochlorid der eingesetzten Base kann gegebenenfalls mechanisch (z.B. durch Filtration) oder durch Ausrühren mit Wasser entfernt werden. Vorzugsweise wird dieses Hydrochlorid nicht abgetrennt.

Für die Stufe b) kommen beispielsweise Reaktionstemperaturen im Bereich 5 bis 100°C in Frage. Bevorzugt arbeitet man bei 10 bis 80°C. Bevorzugte Amine der Formel (V) sind Ethylamin, Cyclopropylamin, 2,4-Difluoranilin, Aminopropanol und Fluorcyclopropylamin.

In die Stufe b) wird im allgemeinen mindestens ein Äquivalent Amin pro Mol Ester der Formel (IV) eingesetzt. Bevorzugt beträgt diese Menge 1 bis 1,3 Äquivalente. Größere Mengen sind nicht kritisch, aber unwirtschaftlich.

Das freigesetzte Dialkylamin, vorzugsweise Dimethyl- oder Diethylamin, wird vorzugsweise aus dem Reaktionsgemisch entfernt, beispielsweise durch Zusatz eines Äquivalents Säure und mechanische Abtrennung, z.B. durch Filtration, oder durch Ausrühren mit Wasser. Gegebenenfalls kann man das in der Stufe a) anfallende Hydrochlorid hier mit abtrennen. Man kann das freigesetzte Dialkylamin beispielsweise auch durch Abdestillieren bei niedrigen Temperaturen aus dem Reaktionsgemisch entfernen.

Für die Stufe c) kommen beispielsweise Reaktionstemperaturen im Bereich 50 bis 200°C in Frage. Die jeweils optimale Reaktionstemperatur hängt vom Substitutionsmuster ab und kann durch routinemäßige Vorversuche leicht ermittelt werden.

Als Basen für die Stufe c) kommen z.B. Kaliumcarbonat, Natriumcarbonat, Natriumhydrid und Natrium-tert.-butylat in Frage. Bevorzugt ist Kaliumcarbonat. Bezogen auf 1 mol der Verbindung der Formel (VI) kann man z.B. 1 bis 4 mol-Äquivalente der Base einsetzen. Vorzugsweise beträgt diese Menge 1,1 bis 1,5 mol-Äquivalente.

Beim Einsatz von Kalium- oder Natriumcarbonat ist es vorteilhaft, das freiwerdende Reaktionswasser zu entfernen, z.B. über einen Wasserabscheider.

Die Stufe c) kann man gegebenenfalls in Gegenwart eines Phasentransferkatalysators durchführen. Geeignet sind beispielsweise Tetraalkylammoniumhalogenide.

Die Isolierung des Esters der Formel (I) mit R¹ = C₁-C₄-Alkyl kann beispielsweise wie folgt erfolgen: Zunächst destilliert man einen Teil des Lösungsmittels ab, z.B. 40 bis 60 Gew.-%, setzt dann Wasser zu, wobei im allgemeinen der Ester auszufallen beginnt, destilliert dann das restliche Lösungsmittel ab, trennt dann den Ester z.B. durch Filtration ab, wäscht ihn mit einem Alkohol, z.B. einem C₁-C₄-Alkylalkohol, und trocknet ihn anschließend im Vakuum.

Die Esterverseifung zur Herstellung von Säuren der Formeln (I) mit R¹ = Wasserstoff aus Estern der Formel (I) mit R¹ = C₁-C₄-Alkyl kann nach allgemein üblichen Methoden in einem sauren oder in einem alkalischen Medium durchgeführt werden. Liegen basenempfindliche Ester der Formel (I) vor, so ist es natürlich bevorzugt, die Ester in einem sauren Medium zu verseifen.

Für die Abtrennung und Isolierung von Säuren der Formel (I) kann man z.B. Essigsäure, Schwefelsäure oder Salzsäure zugeben. Die Abtrennung der ausgefallenen Säure kann z.B. durch Filtration erfolgen.

Es ist ein wesentliches Merkmal des erfindungsgemäßen Verfahrens, daß man die nach Durchführung der Stufen a) und b) erhaltenen Zwischenprodukte der Formeln (IV) und (VI) nicht isoliert.

Es ist auch ein wesentliches Merkmal des erfindungsgemäßen Verfahrens, daß man die Stufen a) bis c) ohne Wechsel des Lösungsmittels im gleichen, wenig polaren bis unpolaren Lösungsmittel durchführt.

Geeignete Lösungsmittel sind beispielsweise Alkylbenzole, insbesondere solche, die 1 bis 3 C₁-C₄-Alkylgruppen pro Molekül enthalten, Halogenbenzole, insbesondere solche, die 1 bis 2 Halogenatome, vorzugsweise Chloratome, pro Molekül enthalten, Halogenalkylbenzole, insbesondere solche, die 1 bis 2 Halogenatome, vorzugsweise Chloratome, und 1 bis 2 C₁-C₄-Alkylgruppen pro Molekül enthalten, alicyclische Kohlenwasserstoffe, insbesondere solche, die 5 bis 7 Ring-C-Atome enthalten und gegebenenfalls mit 1 bis 2 C₁-C₄-Alkylgruppen substituiert sind, offenkettige, gesättigte oder ungesättigte Kohlenwasserstoffe, insbesondere solche, die geradkettig oder verzweigt sind und 5 bis 18 C-Atome enthalten und beliebige Mischungen solcher Lösungsmittel.

Bei den Lösungsmitteln ist darauf zu achten, daß man solche auswählt, deren Siedepunkt bei Normaldruck oberhalb der vorgesehenen Reaktionstemperatur liegt oder, im Falle von Reaktionstemperaturen oberhalb des Siedepunkts bei Normaldruck des vorgesehenen Lösungsmittels darauf, daß man druckfeste, geschlossene Apparaturen verwendet. Liegt der Siedepunkt bei Normaldruck des Lösungsmittels weit oberhalb der vorgesehenen Reaktionstemperatur, so kann auch unter vermindertem Druck gearbeitet werden.

Einzelbeispiele für Lösungsmittel sind: Toluol, Xylole, Mesitylen, Ethylbenzol, Diethybenzole, Isopropylbenzol, Chlorbenzol, Dichlorbenzole, Chlortoluole, Cyclohexan und Kohlenwasserstoffgemische, die mindestens 80 Gew.-% eines oder mehrerer geradkettiger oder verzweigter C₆- bis C₁₂-Kohlenwasserstoffe enthalten.

Bevorzugte Lösungsmittel sind Toluol, Xylole, Mesitylen, Isopropylbenzol, Chlorbenzol und Dichlorbenzole.

Pro Mol Säurechlorid der Formel (II) kann man beispielsweise 300 bis 1000 ml Lösungsmittel einsetzen. Bevorzugt liegt diese Menge bei 400 bis 800 ml. Größere Lösungsmittelmengen sind nicht kritisch, aber unwirtschaftlich.

Das erfindungsgemäße Verfahren hat die Vorteile, daß drei Reaktionsstufen ohne Isolierung der Zwischenstufen und ohne Wechsel des Lösungsmittels durchgeführt werden können und, daß höhere Ausbeuten erreicht werden, als gemäß dem Stand der Technik. Die erzielbaren Ausbeuten liegen bei über 80 % der Theorie, häufig bei über 85 % der Theorie. Das bedeutet, das erfindungsgemäße Verfahren ist auf verfahrenstechnisch einfache Weise und besonders effektiv durchführbar, weil der Aufwand für die Abtrennung und Entsorgung bzw. Aufbereitung eines zweiten Lösungsmittels sowie für die Isolierung und Trocknung von Zwischenprodukten entfällt und trotzdem höhere Ausbeuten als bisher erzielt werden.

Diese erzielbaren Vorteile sind außerordentlich überraschend, denn bisher wurde zumindest für die Cyclisierungsreaktion (Stufe c) der Einsatz von polaren Lösungsmitteln für unabdingbar gehalten.

Besonders bevorzugte Verbindungen, die nach dem erfindungsgemäßen Verfahren aus den entsprechenden Verbindungen der Formeln (II), (III) und (V) hergestellt werden können sind:
1-Cyclopropyl-7-chlor-6-fluor-1,4-dihydro-4-oxo-3-chinolin-carbonsäure,
1-Cyclopropyl-6,7,8-trifluor-1,4-dihydro-4-oxo-3 -chinolin-carbonsäureethylester,
1-Cyclopropyl-6,7-difluor-1,4-dihydro-4-oxo-3-chinolin-carbonsäureethylester,
1-Cyclopropyl-6,7-difluor-8-methoxy-1,4-dihydro-4-oxo-3-chinolin-carbonsäureethylester,
1-Cyclopropyl-6,7-difluor-8-cyano-1,4-dihydro-4-oxo-3-chinolin-carbonsäureethylester,
1-(2-Fluor)cyclopropyl-6,7-difluor-1,4-dihydro-4-oxo-3-chinolin-carbonsäureethylester,
1 -Cyclopropyl-8-chlor-6,7-difluor-1,4-dihydro-4-oxo-3-chinolin-carbonsäureethylester,
1-Ethyl-6,7,8-trifluor-1,4-dihydro-4-oxo-3-chinolin-carbonsäureethylester,
7-Chlor-1-(2,4-difluorphenyl)-6-fluor-1,4-dihydro-4-oxo-1,8-naphthyridon-3-carbonsäureethylester,
7-Chlor-1-cyclopropyl-6-fluor-1,4-dihydro-4-oxo-1,8-naphthyridon-3-carbonsäureethylester,
1-Cyclopropyl-7-chlor-6-fluor-1,4-dihydro-4-oxo-3 -chinolin-carbonsäureethylester und
9,10-Difluor-2,3 -dihydro-3 -methyl-7-oxo-7H-pyrido( 1,2,3-de)(1,4)benzoxazin-6-carbonsäureethylester.

Ein spezieller Aspekt der vorliegenden Erfindung ist ein Verfahren zur Cyclisierung eines (Het-)Aroylacrylesters der Formel (VI) 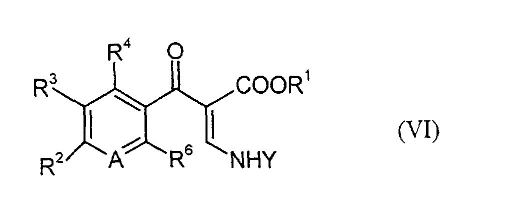 in der
- R¹: für C₁-C₄-Alkyl,
- R²: für Halogen,
- R³: für Halogen,
- R⁴: für Wasserstoff, Halogen oder Nitro,
- R⁶: für Halogen,
- Y: für C₁-C₆-Alkyl, 2-Fluorethyl, Cyclopropyl, Fluorcyclopropyl, Isopropyl, 4-Fluorphenyl oder 2,4-Difluorphenyl und
- A: für Stickstoff oder C-R⁵ mit R⁵ = Wasserstoff, Methyl, Methoxy, Halogen, Nitro oder Cyano stehen,
wobei Y und R⁵ auch gemeinsam -CH₂-CH₂-O- oder -CH(CH₃)-CH₂-O- bedeuten können, wobei die endständige CH₂- bzw. die CH(CH₃)-Gruppe an das Stickstoffatom gebunden ist,
in Gegenwart einer Base unter Bildung eines Esters der Formel (I) 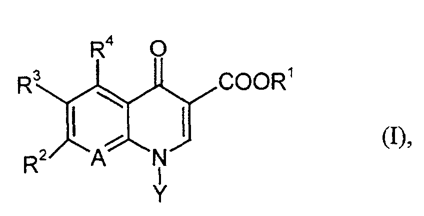 in der die verwendeten Symbole die oben bei Formel (VI) angegebene Bedeutung haben,
das dadurch gekennzeichnet ist, daß man in Gegenwart eines wenig polaren bis unpolaren Lösungsmittels arbeitet.

Nähere Einzelheiten dieses Verfahrens sind weiter oben beschrieben.

Bevorzugte wenig polare bis unpolare Lösungsmittel sind: Alkylbenzole, Halogenbenzole, Halogenalkylbenzole, alicyclische Kohlenwasserstoffe, offenkettige Kohlenwasserstoffe und beliebige Mischungen solcher Lösungsmittel.

### Beispiele

### Beispiel 1

Zu einer Lösung aus 380g Dichlorbenzol (Isomerengemisch), 110 g N,N-Dimethylaminoacrylsäureethylester und 77 g Triethylamin wurden bei 70°C 160 g 2,4-Dichlor-5-fluorbenzoylchlorid innerhalb von 50 Minuten zugetropft. Anschließend wurde 2 Stunden bei 70°C nachgerührt und auf Raumtemperatur abgekühlt. 51 g Essigsäure wurden bei Raumtemperatur zugesetzt und wieder auf 70°C aufgeheizt. Danach wurden 45 g Cyclopropylamin bei 70°C zugetropft, anschließend die Reaktionsmischung mit 100 ml Wasser versetzt und die sich bildende organische Phase abgetrennt. Die organische Phase wurde zu einer Mischung von 59 g Kaliumcarbonat und 190 g Dichlorbenzol (Isomerengemisch) bei 180 bis 184°C zudosiert. Das freiwerdende Reaktionswasser wurde über einen Wasserabscheider abgetrennt. Nach Beendigung der Wasserabscheidung wurde auf 80°C abgekühlt und bei einem Druck von 40 mbar 340 ml Dichlorbenzol abdestilliert. Anschließend wurden 80 g 35 %ige wäßrige Natronlauge und 350 g Wasser zugesetzt und das restliche Dichlorbenzol abdestilliert. Nach Zugabe von 180 g Essigsäure und 100 g Wasser wurde das Produkt abgesaugt, der isolierte Feststoff 3 mal mit je 150 ml Wasser und 3 mal mit je 200 ml Isopropanol gewaschen. Nach dem Trocknen im Vakuum bei 60°C wurden 173 g 1-Cyclopropyl-7-chlor-6-fluor-1,4-dihydro-4-oxo-3-chinolin-carbonsäure erhalten. Das entspricht einer Ausbeute von 87 % der Theorie.

### Beispiel 2

Ein Gemisch aus 380 g Xylol (Isomerengemisch), 110 g N,N-Dimethylaminoacrylsäureethylester und 77,4 g Triethylamin wurden vorgelegt und bei 70°C 160 g 2,4-Dichlor-5-fluorbenzoylchlorid innerhalb von 60 Minuten zugetropft. Anschließend wurde 2 Stunden bei 70°C nachgerührt und auf Raumtemperatur abgekühlt. 51 g Essigsäure wurden dann bei Raumtemperatur zugesetzt und wieder auf 70°C aufgeheizt. Danach wurden 45 g Cyclopropylamin bei 70°C zugetropft. Zur Reaktionsmischung wurden dann 100 ml Wasser gegeben, 15 Minuten gerührt und die sich bildende organische Phase abgetrennt. Die organische Phase wurde zu einer Mischung von 89 g Kaliumcarbonat und 190 g Xylol (Isomerengemisch) bei 140 bis 142°C zudosiert. Das freiwerdende Reaktionswasser wurde über einen Wasserabscheider abgetrennt. Nach Beendigung der Wasserabscheidung wurde auf 80°C abgekühlt und bei einem Druck von 40 mbar Xylol abdestilliert. Anschließend wurden 80 g 45 %ige wäßrige Natronlauge und 350 g Wasser zugesetzt und das restliche Xylol abdestilliert. Nach Zugabe von 180 g Essigsäure und 100 g Wasser wurde das Produkt abgesaugt und der Feststoff 3 mal mit je 150 ml Wasser und 3 mal mit je 200 ml Isopropanol gewaschen. Nach dem Trocknen im Vakuum bei 60°C wurden 170 g 1-Cyclopropyl-7-chlor-6-fluor-1,4-dihydro-4-oxo-3-chinolin-carbonsäure erhalten. Das entspricht einer Ausbeute von 86 % der Theorie.

### Beispiel 3

380 g Chlorbenzol, 110 g N,N-Dimethylaminoacrylsäureethylester und 77,4 g Triethylamin wurden vorgelegt und bei 70°C 160 g 2,4-Dichlor-5-fluorbenzoylchlorid innerhalb von 60 Minuten zugetropft. Anschließend wurde 2 Stunden bei 70°C nachgerührt, dann auf Raumtemperatur abgekühlt. Nunmehr wurden 51 g Essigsäure bei Raumtemperatur zugesetzt und wieder auf 70°C aufgeheizt. Danach wurden 45 g Cyclopropylamin bei 70°C zugetropft. Zur Reaktionsmischung wurden 100 ml Wasser zugesetzt, 15 Minuten gerührt und die sich bildende organische Phase abgetrennt. Die wäßrige Phase wurde mit 50 ml Chlorbenzol extrahiert und die vereinigten organischen Phasen zu einer Mischung von 119 g Kaliumcarbonat, 1 g Tributylammoniumbromid und 190 g Chlorbenzol bei 131°C zudosiert. Das freiwerdende Reaktionswasser wurde über einen Wasserabscheider abgetrennt. Nach Beendigung der Wasserabscheidung wurde auf 20°C abgekühlt und der ausgefallene Feststoff über eine Filternutsche abgesaugt. Der Feststoff wurde dann 3 mal mit je 200 ml Isopropanol gewaschen. Nach dem Trocknen im Vakuum bei 60°C wurden 186 g 1-Cyclopropyl-7-chlor-6-fluor-1,4-dihydro-4-oxo-3-chinolin-carbonsäureethylester erhalten. Das entspricht einer Ausbeute von 86 % der Theorie.

### Beispiel 4

Zu einer Lösung von 270 g Toluol, 189,8 g N,N-Dimethylaminoacrylsäureethylester und 144,2 g Triethylamin wurden bei 45°C 280 g 2,3,4,5-Tetrafluorbenzoylchlorid innerhalb von 60 Minuten zugetropft. Anschließend wurde 1 Stunde bei 50°C nachgerührt, dann auf Raumtemperatur abgekühlt. Nunmehr wurden 95,2 g Essigsäure bei Raumtemperatur zugesetzt und dann, 75,2 g Cyclopropylamin bei 20 bis 30°C zugetropft. Danach wurden der Reaktionsmischung wurden 200 ml Wasser zugesetzt und die sich bildende organische Phase abgetrennt. Die wäßrige Phase wurde mit 82 g Toluol extrahiert und die vereinigten organischen Phasen zu einer Mischung von 110 g Kaliumcarbonat, und 404 g Toluol bei 111°C zudosiert. Das freiwerdende Reaktionswasser wurde über einen Wasserabscheider abgetrennt. Nach Beendigung der Wasserabscheidung wurde auf 60°C abgekühlt und 1280 g Wasser zugesetzt. Bei einer Temperatur von 40°C und einem Druck von 100 mbar wurde das Toluol abdestilliert. Die Suspension wurde auf 20°C abgekühlt und über eine Filternutsche abgesaugt. Der Feststoff wurde dann 3 mal mit je 200 ml Wasser und 3 mal mit je 250 ml Isopropanol gewaschen und anschließend im Vakuum bei 50°C getrocknet. Es wurden 374 g 1-Cyclopropyl-6,7,8-trifluor-1,4-dihydro-4-oxo-3-chinolin-carbonsäureethylester erhalten. Das entspricht einer Ausbeute von 91 % der Theorie.

### Beispiel 5

Zu einer Lösung von 202 g Toluol, 94,9 g N,N-Dimethylaminoacrylsäureethylester und 72,1 g Triethylamin wurden bei 45°C 140 g 2,3,4,5-Tetrafluorbenzoylchlorid innerhalb von 60 Minuten zugetropft. Anschließend wurde 1 Stunde bei 43°C nachgerührt und dann auf Raumtemperatur abgekühlt. Nunmehr wurden 37,6 g Cyclopropylamin bei 20 bis 30°C zugetropft und 1 Stunde gerührt. Anschließend wurde bei einem Druck von 80 mbar das Dimethylamin abdestilliert. Zur Reaktionsmischung wurden 100 ml Wasser zugesetzt und die sich bildende organische Phase abgetrennt. Die wäßrige Phase wurde mit 41 g Toluol extrahiert und die vereinigten organischen Phasen zu einer Mischung von 55 g Kaliumcarbonat und 202 g Toluol bei 110°C zudosiert. Das freiwerdende Reaktionswasser wurde über einen Wasserabscheider abgetrennt. Nach Beendigung der Wasserabscheidung wurde auf 60°C abgekühlt und 640 g Wasser zugesetzt. Bei einer Temperatur von 40°C und einem Druck von 100 mbar wurde das Toluol abdestilliert. Die Suspension wurde auf 20°C abgekühlt und über eine Filternutsche abgesaugt. Der erhaltene Feststoff wurde 3 mal mit je 150 ml Wasser und 3 mal mit je 150 ml Isopropanol gewaschen und anschließend bei 50°C im Vakuum getrocknet. So wurden 172 g 1-Cyclopropyl-6,7,8-trifluor-1,4-dihydro-4-oxo-3-chinolin-carbonsäureethylester erhalten. Das entspricht einer Ausbeute von 84 % der Theorie.

### Beispiel 6

Es wurde analog Beispiel 2 verfahren, jedoch wurde anstelle von Xylol Isopropylbenzol eingesetzt und die Cyclisierung bei 156 bis 158°C durchgeführt. Nach dem Trocknen bei 60°C im Vakuum wurden 177 g 1-Cyclopropyl-7-chlor-6-fluor-1,4-dihydro-4-oxo-3-chinolin-carbonsäure erhalten. Das entspricht einer Ausbeute von 89 % der Theorie.

### Beispiel 7

Es wurde analog Beispiel 2 gearbeitet, jedoch wurde anstelle von Xylol Mesitylen verwendet und die Cyclisierung bei 166 bis 168°C durchgeführt. Nach dem Trocknen bei 60°C im Vakuum wurden 174 g 1-Cyclopropyl-7-chlor-6-fluor-1,4-dihydro-4-oxo-3-chinolin-carbonsäure erhalten. Das entspricht einer Ausbeute von 88 % der Theorie.

### Beispiel 8

272 g Toluol, 111 g N,N-Dimethylaminoacrylsäureethylester und 85 Triethylamin wurden vorgelegt und bei 50 bis 55°C innerhalb von 60 Minuten 156 2,4,5-Trifluorbenzoylchlorid zugetropft. Anschließend wurde 2 Stunden bei 55°C nachgerührt, dann auf Raumtemperatur abgekühlt. Nun wurden 56 g Essigsäure zugesetzt und 48,6 g Cyclopropylamin bei 20 bis 30°C zugetropft. Zur Reaktionsmischung wurden dann 250 ml Wasser zugefügt, 15 Minuten gerührt und die organische Phase abgetrennt. Die organische Phase wurde zu einer Mischung von 65 g Kaliumcarbonat und 240 g Toluol bei 110°C zudosiert. Das freiwerdende Reaktionswasser wurde über einen Wasserabscheider abgetrennt. Nach Ende der Wasserabscheidung wurde auf 30°C abgekühlt und 500 ml Wasser zugesetzt. Bei einem Druck von 120 bis 180 mbar wurde Toluol abdestilliert. Anschließend wurde auf 20°C abgekühlt und das Produkt abgesaugt. Der isolierte Feststoff wurde drei mal mit je 100 ml Wasser und drei mal mit je 100 ml Isopropanol gewaschen. Nach dem Trocknen bei 50°C im Vakuum wurden 206 g 1-Cyclopropyl-6,7-difluor-1,4-dihydro-4-oxo-3-chinolin-carbonsäureethylester erhalten. Das entspricht einer Ausbeute von 88 % der Theorie.

## Patentansprüche

1. Verfahren zur Herstellung von Chinolon- und Naphthyridoncarbonsäuren und deren Ester der Formel (I) 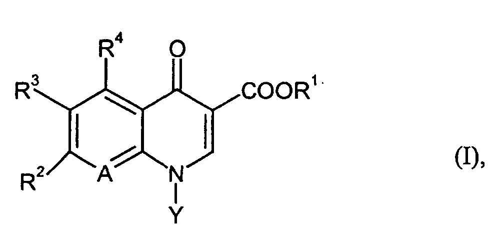 in der
R¹ für Wasserstoff oder C₁-C₄-Alkyl,
R² für Halogen,
R³ für Halogen,
R⁴ für Wasserstoff, Halogen oder Nitro,
Y für C₁-C₆-Alkyl, 2-Fluorethyl, Cyclopropyl, Fluorcyclopropyl, Isopropyl, 4-Fluorphenyl oder 2,4-Difluorphenyl und
A für Stickstoff oder C-R⁵ mit R⁵ = Wasserstoff, Methyl, Methoxy, Halogen, Nitro oder Cyano stehen,
wobei Y und R⁵ auch gemeinsam -CH₂-CH₂-O- oder -CH(CH₃)-CH₂-O- bedeuten können, wobei die endständige CH₂- bzw. die CH(CH₃)-Gruppe an das Stickstoffatom gebunden ist,
**dadurch gekennzeichnet, dass** man
a) ein Benzoyl- oder Nicotinoylchlorid der Formel (II) 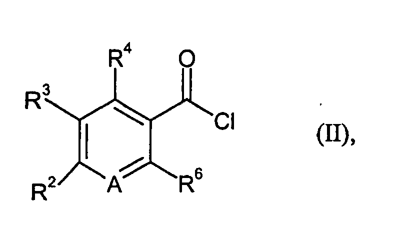 in der
R², R³, R⁴ und A die bei Formel (I) angegebene Bedeutung haben und
R⁶ für Halogen steht,
in Gegenwart einer Base mit einem Aminoacrylsäureester der Formel (III) 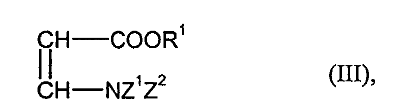 in der
R¹ für C₁-C₄-Alkyl und
Z¹ und Z² unabhängig voneinander fiir C₁-C₄-Alkyl stehen oder zusammen mit dem Stickstoffatom, an das sie gebunden sind, einen 5- bis 6-gliedrigen gesättigten oder ungesättigten Ring bilden, der gegebenenfalls bis zu zwei weitere Heterogruppen aus der Gruppe O-Atome, S-Atome und SO₂-Gruppen enthalten kann,
umsetzt und so einen (Het)-Aroylacrylester der Formel (IV) erhält 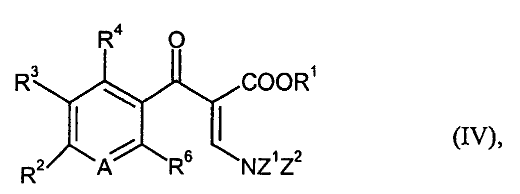 in der
R¹ für C₁-C₄-Alkyl steht und
R², R³, R⁴ und A die bei Formel (I) angegebene,
R⁶ die bei Formel (II) angegebene und
Z¹ und Z² die bei Formel (III) angegebene Bedeutung haben,
b) den (Het-)Aroylacrylester der Formel (IV) mit einem Amin der Formel (V)
H₂N-Y (V),
in der
Y die bei Formel (I) angegebene Bedeutung hat,
einem Aminaustausch unterwirft und so einen (Het-)Aroylacrylester der Formel (VI) erhält 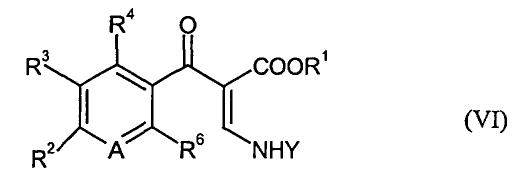 in der
R¹ für C₁-C₄-Alkyl steht und
R², R³, R⁴, Y und A die bei Formel (I) angegebene und
R⁶ die bei Formel (II) angegebene Bedeutung haben,
c) den (Het-)Aroylacrylester der Formel (VI) in Gegenwart einer Base cyclisiert und so einen Chinolon- bzw. Naphthyridonester der Formel (I) erhält, bei dem R¹ für C₁-C₄-Alkyl steht und
d) falls man eine Chinolon- oder Naphthyridoncarbonsäure der Formel (I), in der R¹ für Wasserstoff steht, herstellen möchte, den nach der Stufe c) vorliegenden Ester verseift und die Säure der Formel (I), in der R¹ für Wasserstoff steht, nach Zugabe einer Säure isoliert,
wobei man die Zwischenprodukte der Formeln (IV) und (VI) nicht isoliert und die Stufen a) bis c) in Gegenwart des gleichen Lösungsmittels durchführt, das ausgewählt ist aus der Gruppe Alkylbenzole, Halogenbenzole, Halogenalkylbenzole, alicyclische Kohlenwasserstoffe, offenkettige Kohlenwasserstoffe oder beliebige Mischungen dieser Lösungsmittel.

2. Verfahren nach Anspruch 1, **dadurch gekennzeichnet, dass** man als Lösungsmittel Toluol, Xylole, Mesitylen, Isopropylbenzol, Chlorbenzol oder Dichlorbenzole einsetzt.

3. Verfahren nach Ansprüchen 1 bis 2, **dadurch gekennzeichnet, dass** man pro Mol Säurechlorid der Formel (II) 300 bis 1000 ml Lösungsmittel einsetzt.

4. Verfahren nach Ansprüchen 1 bis 3, **dadurch gekennzeichnet, dass** die in den Formeln (I) bis (VI) verwendeten Symbole folgende Bedeutungen haben:
R¹ in Formel (I) Wasserstoff, in den Formeln (II) bis (VI) Methyl oder Ethyl,
R² Chlor oder Fluor,
R³ Fluor,
R⁴ Wasserstoff, Chlor, Fluor oder Nitro,
R⁶ Fluor oder Chlor,
A C-R⁵ mit R⁵ = Wasserstoff, Methyl, Methoxy, Halogen oder Cyano oder N,
Y Ethyl, Cyclopropyl, Fluorcyclopropyl, 2,4-Difluorphenyl oder zusammen mit R⁵ -CH(CH₃)-CH₂-O- und
Z¹ und Z² Methyl oder Ethyl.

5. Verfahren nach Ansprüchen 1 bis 4, **dadurch gekennzeichnet, dass** man die Stufe a) bei 25 bis 120°C, die Stufe b) bei 5 bis 100°C und die Stufe c) bei 50 bis 200°C durchführt.

6. Verfahren nach Ansprüchen 1 bis 5, **dadurch gekennzeichnet, dass** man in Stufe a) als Basen tertiäre Amine, in Stufe b) als Amine Ethylamin, Cyclopropylamin, 2,4-Difluoranilin, Aminopropanol oder Fluorcyclopropylamin und als Base in Stufe c) Kaliumcarbonat, Natriumcarbonat, Natriumhydrid oder Natrium-tert.-butylat einsetzt.

7. Verfahren zur Cyclisierung eines (Het)-Aroylacrylesters der Formel (VI) 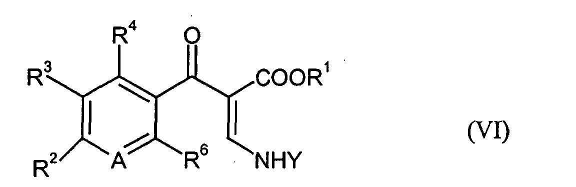 in der
R¹ für C₁-C₄-Alkyl,
R² für Halogen,
R³ für Halogen,
R⁴ für Wasserstoff, Halogen oder Nitro,
R⁶ für Halogen,
Y für C₁-C₆-Alkyl, 2-Fluorethyl, Cyclopropyl, Fluorcyclopropyl, Isopropyl, 4-Fluorphenyl oder 2,4-Difluorphenyl und
A für Stickstoff oder C-R⁵ mit R⁵ = Wasserstoff, Methyl, Methoxy, Halogen, Nitro oder Cyano stehen,
wobei Y und R⁵ auch gemeinsam -CH₂-CH₂-O- oder -CH(CH₃)-CH₂-O- bedeuten können, wobei die endständige CH₂- bzw. die CH(CH₃)-Gruppe an das Stickstoffatom gebunden ist,
in Gegenwart einer Base unter Bildung eines Esters der Formel (I) 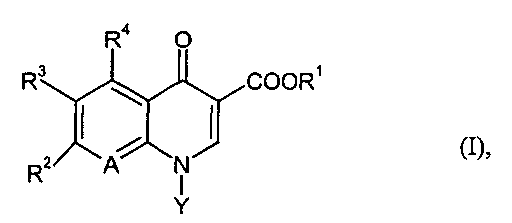 in der die verwendeten Symbole die oben bei Formel (VI) angegebene Bedeutung haben,
**dadurch gekennzeichnet, dass** man in Gegenwart eines Lösungsmittels arbeitet, das ausgewählt ist aus der Gruppe Alkylbenzole, Halogenbenzole, Halogenalkylbenzole, alicyclische Kohlenwasserstoffe, offenkettige Kohlenwasserstoffe oder beliebige Mischungen dieser Lösungsmittel

## Claims

1. Process for preparing quinolone- and naphthyridonecarboxylic acids and esters thereof of the formula (I) 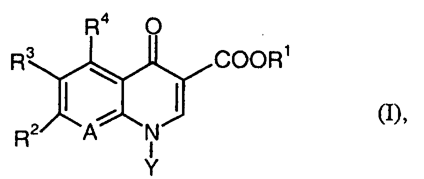 in which
R¹ represents hydrogen or C₁-C₄-alkyl,
R² represents halogen,
R³ represents halogen,
R⁴ represents hydrogen, halogen or nitro,
Y represents C₁-C₆-alkyl, 2-fluoroethyl, cyclopropyl, fluorocyclopropyl, isopropyl, 4-fluorophenyl or 2,4-difluorophenyl and
A represents nitrogen or C-R⁵ where R⁵ = hydrogen, methyl, methoxy, halogen, nitro or cyano,
where Y and R⁵ together may also represent -CH₂-CH₂-O- or -CH(CH₃)-CH₂-O-, where the terminal CH₂- or the CH(CH₃)- group is attached to the nitrogen atom,
**characterized in that**
a) a benzoyl or nicotinoyl chloride of the formula (II) 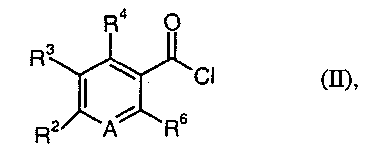 in which
R², R³, R⁴ and A are each as defined under formula (I) and
R⁶ represents halogen,
is reacted in the presence of a base with an aminoacrylic ester of the formula (III) 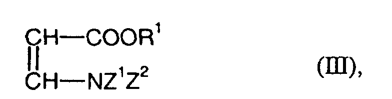 in which
R¹ represents C₁-C₄-alkyl and
Z¹ and Z² independently of one another each represent C₁-C₄-alkyl or together with the linking nitrogen atom form a 5- to 6-membered saturated or unsaturated ring which may optionally contain up to two further hetero groups selected from the group consisting of O atoms, S atoms and SO₂ groups,
thus giving a (het)-aroylacrylic ester of the formula (IV) 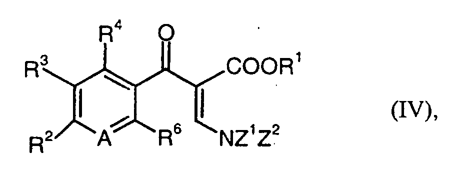 in which
R¹ represents C₁-C₄-alkyl and
R², R³, R⁴ and A are each as defined under formula (I),
R⁶ is as defined under formula (II) and
Z¹ and Z² are each as defined under formula (III),
b) the (het)-aroylacrylic ester of the formula (IV) is subjected to an amine exchange with an amine of the formula (V)
H₂N-Y (V),
in which
Y is as defined under (I),
thus giving a (het)-aroylacrylic ester of the formula (VI) 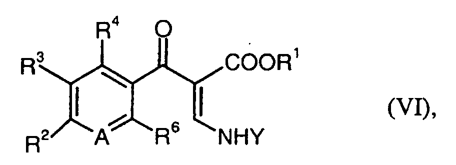 in which
R¹ represents C₁-C₄-alkyl and
R², R³, R⁴, Y and A are each as defined under formula (I) and
R⁶ is as defined under formula (II),
c) the (Het)-aroylacrylic ester of the formula (VI) is cyclized in the presence of a base, thus giving a quinolone or naphthyridone ester of the formula (I) in which R¹ represents C₁-C₄-alkyl and
d) if a quinolone- or naphthyridonecarboxylic acid of the formula (I) is to be prepared in which R¹ represents hydrogen, the ester which is present after step c) is hydrolyzed and the acid of the formula (I) in which R¹ represents hydrogen is isolated after addition of an acid,
where the intermediates of the formulae (IV) and (VI) are not isolated and steps a) to c) are carried out in the presence of the same solvent which is selected from the group comprising alkylbenzenes, halogenobenzenes, halogenoalkylbenzenes, alicyclic hydrocarbons, open-chain hydrocarbons, or any mixtures of these solvents.

2. Process according to Claim 1, **characterized in that** the solvent used is toluene, xylene, mesitylene, isopropylbenzene, chlorobenzene or dichlorobenzene.

3. Process according to Claims 1 and 2, **characterized in that** from 300 to 1000 ml of solvent are employed per mole of acyl chloride of the formula (II).

4. Process according to Claims 1 to 3, **characterized in that** the symbols used in the formulae (I) to (VI) have the following meanings:
R¹, in formula (I), is hydrogen and, in formulae (II) to (VI), is methyl or ethyl,
R² is chlorine or fluorine,
R³ is fluorine,
R⁴ is hydrogen, chlorine, fluorine or nitro,
R⁶ is fluorine or chlorine,
A is C-R⁵ where R⁵ = hydrogen, methyl, methoxy, halogen or cyano or N,
Y is ethyl, cyclopropyl, fluorocyclopropyl, 2,4-difluorophenyl or together with R⁵ -CH(CH₃)-CH₂-O- and
Z¹ and Z² are methyl or ethyl.

5. Process according to Claims 1 to 4, **characterized in that** step a) is carried out at from 25 to 120°C, step b) is carried out at from 5 to 100°C and step c) is carried out at from 50 to 200°C.

6. Process according to Claims 1 to 5, **characterized in that** the base used in step a) is a tertiary amine, the amine used in step b) is ethylamine, cyclopropylamine, 2,4-difluoroaniline, aminopropanol or fluorocyclopropylamine and the base used in step c) is potassium carbonate, sodium carbonate, sodium hydride or sodium tert-butoxide.

7. Process for cyclizing a (het)-aroylacrylic ester of the formula (VI) 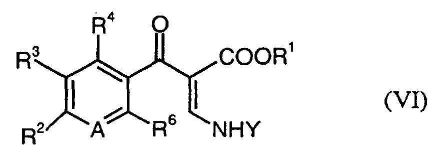 in which
R¹ represents C₁-C₄-alkyl,
R² represents halogen,
R³ represents halogen,
R⁴ represents hydrogen, halogen or nitro,
R⁶ represents halogen,
Y represents C₁-C₆-alkyl, 2-fluoroethyl, cyclopropyl, fluorocyclopropyl, isopropyl, 4-fluorophenyl or 2,4-difluorophenyl and
A represents nitrogen or C-R⁵ where R⁵ = hydrogen, methyl, methoxy, halogen, nitro or cyano,
where Y and R⁵ together may also represent -CH₂-CH₂-O- or -CH(CH₃)-CH₂-O-, where the terminal CH₂- or the CH(CH₃)- group is attached to the nitrogen atom,
in the presence of a base, forming an ester of the formula (I) 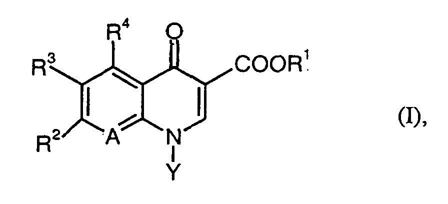 in which the symbols used are each as defined above under formula (VI),
**characterized in that** the process is carried out in the presence of a solvent which is selected from the group comprising alkylbenzenes, halogenobenzenes, halogenoalkylbenzenes, alicyclic hydrocarbons, open-chain hydrocarbons, or any mixtures of these solvents.

## Revendications

1. Procédé pour la préparation d'acides quinolone- et naphtyridone-carboxyliques et de leurs esters répondant à la formule (I) 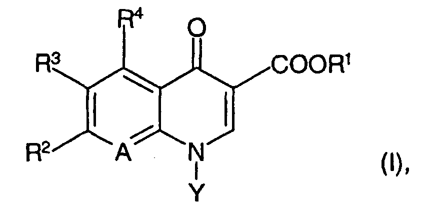 dans laquelle
R¹ représente l'hydrogène ou un groupe alkyle en C₁ à C₄,
R² représente un halogène,
R³ représente un halogène,
R⁴ représente l'hydrogène, un halogène ou un groupe nitro,
Y représente un groupe alkyle en C₁ à C₆, 2-fluoroéthyle, cyclopropyle, fluorocyclopropyle, isopropyle, 4-fluorophényle ou 2,4-difluorophényle, et
A représente l'azote ou le groupe C-R⁵ pour lequel R⁵ = hydrogène, méthyle, méthoxy, halogène, nitro ou cyano,
Y et R⁵ pouvant également former ensemble -CH₂-CH₂-O- ou -CH(CH₃)-CH₂-O-, dont le groupe terminal CH₂ ou CH(CH₃) respectivement est relié à l'atome d'azote,
ce procédé se caractérisant en ce que,
a) on fait réagir un chlorure de benzoyle ou de nicotinoyle de formule (II) 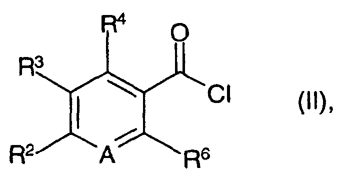 dans laquelle
R², R³, R⁴ et A ont les significations indiquées en référence à la formule (I) et
R⁶ représente un halogène,
en présence d'une base, avec un ester aminoacrylique de formule (III), 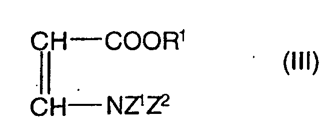 dans laquelle
R¹ représente un groupe alkyle en C₁ à C₄, et
Z¹ et Z² représentent chacun, indépendamment l'un de l'autre, un groupe alkyle en C₁ à C₄ ou forment ensemble et avec l'atome d'azote auquel ils sont reliés un noyau saturé ou insaturé de 5 ou 6 chaînons, qui peut contenir le cas échéant jusqu'à deux autres motifs hétérogènes choisis parmi les atomes d'oxygène, les atomes de soufre et les groupes SO₂,
ce qui donne un ester (hétéro)-aroylacrylique de formule (IV) 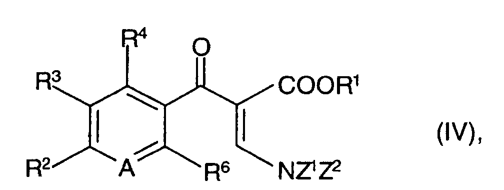 dans laquelle
R¹ représente un groupe alkyle en C₁ à C₄, et
R², R³, R⁴ et A ont les significations indiquées en référence à la formule (I),
R⁶ a les significations indiquées en référence à la formule (II) et
Z¹ et Z² ont les significations indiquées en référence à la formule (III),
b) sur l'ester (hétéro)-aroylacrylique de formule (IV), on procède à un échange d'amine à l'aide d'une amine de formule (V),
H₂N-Y (V),
dans laquelle
Y a les significations indiquées en référence à la formule (I),
ce qui donne un ester (hétéro)-aroylacrylique de formule (VI) 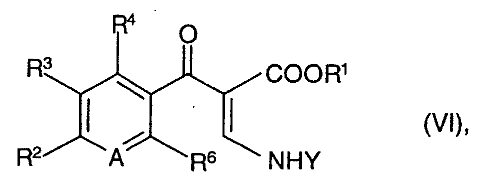 dans laquelle
R¹ représente un groupe alkyle en C₁ à C₄, et
R², R³, R⁴, Y et A ont les significations indiquées en référence à la formule (I), et
R⁶ a les significations indiquées en référence à la formule (II),
c) on cyclise l'ester (hétéro)-aroylacrylique de formule (VI) en présence d'une base, ce qui donne un ester quinolone- ou respectivement naphtyridone-carboxylique de formule (I) dans laquelle R¹ représente un groupe alkyle en C₁ à C₄, et
d) lorsqu'on désire obtenir un acide quinolone- ou naphtyridone-carboxylique de formule (I) dans laquelle R¹ représente l'hydrogène, on saponifie l'ester obtenu au stade c), puis on isole l'acide de formule (I) dans laquelle R¹ représente l'hydrogène après addition d'un acide,
les produits intermédiaires de formules (IV) et (VI) n'étant pas isolés et les stades a) à c) étant exécutés en présence du même solvant choisi dans le groupe consistant en les alkylbenzènes, les halogénobenzènes, les halogèno-alkylbenzènes, les hydrocarbures alicycliques, les hydrocarbures acycliques et les mélanges quelconques de ces solvants.

2. Procédé selon la revendication 1, **caractérisé en ce que** le solvant utilisé est choisi parmi le toluène, les xylènes, le mésitylène, l'isopropylbenzène, le chlorobenzène et les dichlorobenzènes.

3. Procédé selon les revendications 1 à 2, **caractérisé en ce que** l'on utilise de 300 à 1000 ml de solvant par mole du chlorure d'acide de formule (II).

4. Procédé selon les revendications 1 à 3, **caractérisé en ce que** les symboles des formules (I) à (VI) ont les significations suivantes :
R¹ : dans la formule (I) : l'hydrogène ; dans les formules (II) à (VI) : méthyle ou éthyle,
R² : le chlore ou le fluor,
R³ : le fluor,
R⁴ : l'hydrogène, le chlore, le fluor ou un groupe nitro,
R⁶ : le fluor ou le chlore,
A : C-R⁵ avec R⁵ = hydrogène, méthyle, méthoxy, halogène ou cyano ou N,
Y : éthyle, cyclopropyle, fluorocyclopropyle, 2,4-difluorophényle ou bien, avec R⁵,-CH(CH₃)-CH₂-O- et
Z¹ et Z² : méthyle ou éthyle.

5. Procédé selon les revendications 1 à 4, **caractérisé en ce que**, au stade a), la température va de 25 à 120°C, au stade b) de 5 à 100°C et au stade c) de 50 à 200°C.

6. Procédé selon les revendications 1 à 5, **caractérisé en ce que**, au stade a), on utilise en tant que bases des amines tertiaires, au stade b) en tant qu'amines l'éthylamine, la cyclopropylamine, la 2,4-difluoroaniline, l'aminopropanol ou la fluorocyclopropylamine et au stade c), en tant que bases le carbonate de potassium, le carbonate de sodium, l'hydrure de sodium ou le tert-butylate de sodium.

7. Procédé pour la cyclisation d'un ester (hétéro)-aroylacrylique de formule (VI) 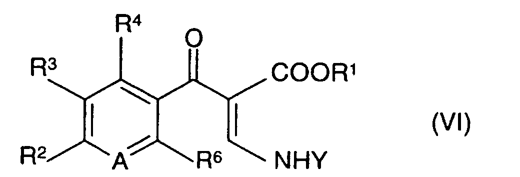 dans laquelle
R¹ représente un groupe alkyle en C₁ à C₄,
R² représente un halogène,
R³ représente un halogène,
R⁴ représente l'hydrogène, un halogène ou un groupe nitro,
R⁶ représente un halogène,
Y représente un groupe alkyle en C₁ à C₆, 2-fluoroéthyle, cyclopropyle, fluorocyclopropyle, isopropyle, 4-fluorophényle ou 2,4-difluorophényle, et
A représente l'azote ou le groupe C-R⁵ avec R⁵ = hydrogène, méthyle, méthoxy, halogène, nitro ou cyano,
Y et R⁵ pouvant également représenter ensemble -CH₂-CH₂-O- ou -CH(CH₃)-CH₂-O-, dont le groupe terminal CH₂ ou CH(CH₃) respectivement est relié à l'atome d'azote,
en présence d'une base, avec formation d'un ester de formule (I) 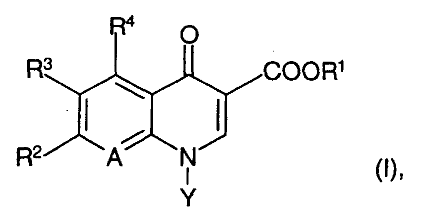 dans laquelle les symboles ont les significations indiquées ci-dessus en référence à la formule (VI),
**caractérisé en ce que** l'on opère en présence d'un solvant choisi dans le groupe formé par les alkylbenzènes, les halogénobenzènes, les halogénoalkylbenzènes, les hydrocarbures alicycliques, les hydrocarbures acycliques et les mélanges quelconques de ces solvants.
